# EUROPEAN PATENT SPECIFICATION

(11) **EP 3 934 702 B1**
(45) Date of publication and mention of the grant of the patent: **08.05.2024**
(21) Application number: 20766269.3
(22) Date of filing: 06.03.2020
(51) Int. Cl.: A61K 51/04, A61P 25/28, C07B 59/00, C07D 417/04, G01N 33/60, C12Q 1/48, A61K 101/02, A61K 101/00

(54) **[ 18F]-LABELED BENZOTHIAZOLE DERIVATIVE AS PET RADIOTRACER**
[18F]-MARKIERTES BENZOTHIAZOLDERIVAT ALS PET-RADIOTRACER
DÉRIVÉ DE BENZOTHIAZOLE MARQUÉ AU [18F] EN TANT QUE RADIOTRACEUR TEP

(30) Priority: 07.03.2019 US 201962814962 P
(43) Date of publication of application: 12.01.2022
(73) Proprietor: 1st Biotherapeutics, Inc., Seongnam-si, Gyeonggi-do 13493 (KR)
(72) Inventor: LEE, Jinhwa, Yongin-si Gyeonggi-do 16876 (KR); JO, Suyeon, Yongin-si Gyeonggi-do 16832 (KR)
(74) Representative: Haseltine Lake Kempner LLP
(86) International application number: PCT/IB2020/051970
(87) International publication number: WO 2020/178795

(56) References cited:
- WO-A1-2010/100144
- WO-A2-2011/045415
- WO-A2-2011/045415
- WO-A2-2012/080284
- KR-A- 20140 107 153
- US-A1- 2010 075 965
- US-A1- 2010 197 682
- US-A1- 2011 178 070

## Description

### CROSS-REFERENCE TO RELATED APPLICATIONS

This application claims the benefits of, and priority to, U.S. provisional application serial numbers 62/814,962 filed on 7 March 2019.

### FIELD

The present disclosure generally relates to fluorine-18 labeled benzothiazole compounds as positron emission tomography (PET) tracers for imaging enzyme inhibitory activity comprising the compound and methods of using the compounds for diagnosis and imaging.

### BACKGROUND

Positron emission tomography (PET) is a nuclear imaging methodology that detects pairs of gamma rays emitted indirectly by a positron-producing radionuclide. Radiotracers are used in PET as diagnostic tools and to image tissue concentration of molecules of interest.

The development of molecular imaging biomarkers is closely related to the development of therapeutic agents. Among the potential targets, the tyrosine kinase c-abl is tightly regulated non-receptor protein tyrosine kinase involved in a wide range of cellular processes, including growth, survival and stress response (Nat Rev Mol Cell Biol, 2004, 5:33-44) and c-abl is involved in regulation of several cellular processes and has implicated in the development of the central nervous system by controlling neurogenesis. More recently, increasing evidence from various experimental model systems has also revealed that c-abl is activated in neurodegenerative disease such as Alzheimer's disease, Parkinson's disease, Niemann-Pick type C diseases and tauopathies. (Human Molecular Genetics, 2014, Vol. 23, No.11)

Recent studies have revealed a critical role for c-abl in Parkinson's disease (PD), such as induction of alpha-synuclein aggregation through its direct phosphorylation, inactivation of parkin and induction of neuroinflammation. The expression level of total c-abl as well as activated c-abl measured by Y412 and Y214 phosphorylated forms are known to be upregulated in PD pathogenesis in various animal models and more importantly in human specimen such as post-mortem whole brain or striatum samples from PD patients. WO2011045415 discloses PET imaging agents targeting amyloids hence useful for diagnostics of neurodegenerative diseases e.g. Alzheimer's disease (AD)(page 1, line 5 - page 2, line 30). In particular, WO2011045415 discloses a benzothiazole compound labeled with fluorine-18 (2-(2-[18F]fluoropyridin-4-yl)-6-hydroxybenzothiazole) (page 38, line 15 - line 20).

However, it has not been explored yet whether the level of c-abl in substantia nigra or striatum in live PD patients is upregulated or not. It would be critical to understand the pathophysiological roles for c-abl in PD and its functional link with disease stages and symptomatic status by investigating c-abl expression level from the brain of PD patients.

The present invention provides a new fluorine-18 radiolabeled compound selective for targeting c-abl as a PET tracer for in vitro and in vivo imaging study.

The IC₅₀'s for c-abl catalytic inhibition of each cold compounds, Formula IIA and IIB are in the range of 5 nM to 20 nM. We utilize the corresponding cold compounds to synthesize ¹⁸F radiotracers for PET.

### SUMMARY

The present disclosure provides a compound having c-abl kinase inhibitory activity, a composition comprising the compound and a method useful to treat a neurodegenerative disease as defined in the claims.

In an embodiment, the compound is a compound of Formula (I): 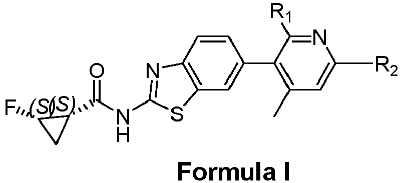 wherein R₁ and R₂ are as defined as below.

In another embodiment, the present invention provides an enantiomer, diastereomer or racemate of the compound of Formula (I) or a pharmaceutically acceptable salt thereof.

In yet another embodiment, the present disclosure provides a method of utilizing the compound for visualizing brain abnormalities, cell death and neural injury with the diverse landscape of neurological imaging.

### DETAILED DESCRIPTION

The following description is merely exemplary in nature.

### Definitions

As used herein, the term "pharmaceutically acceptable" means suitable for use in pharmaceutical preparations, generally considered as safe for such use, officially approved by a regulatory agency of a national or state government for such use, or being listed in the U.S. Pharmacopoeia or other generally recognized pharmacopoeia for use in animals, and more particularly in humans.

As used herein, the term "pharmaceutically acceptable carrier" refers to a diluent, adjuvant, excipient, or carrier, or other ingredient which is pharmaceutically acceptable and with which a compound of the invention is administered.

As used herein, the term "pharmaceutically acceptable salt" refers to a salt which may enhance desired pharmacological activity. Examples of pharmaceutically acceptable salts include acid addition salts formed with inorganic or organic acids, metal salts and amine salts. Examples of acid addition salts formed with inorganic acids include salts with hydrochloric acid, hydrobromic acid, sulfuric acid, nitric acid and phosphoric acid. Examples of acid addition salts formed with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxy-benzoyl)-benzoic acid, cinnamic acid, mandelic acid, methanesulfonic acid, ethanesulfonic acid, 1,2-ethanedisulfonic acid, 2-hydroxyethane-sulfonic acid, benzenesulfonic acid, p-chlorobenzenesulfonic acid, 2-naphthalenesulfonic acid, p-toluenesulfonic acid, camphorsulfonic acid, 4-methyl-bicyclo[2.2.2]oct-2-ene1-carboxylic acid, gluco-heptonic acid, 4,4'-methylenebis(3-hydroxy-2-naphthoic) acid, 3-phenylpropionic acid, trimethyl-acetic acid, tertiary butylacetic acid, lauryl sulfuric acid, gluconic acid, glutamic acid, hydroxy-naphthoic acids, salicylic acid, stearic acid and muconic acid. Examples of metal salts include salts with sodium, potassium, calcium, magnesium, aluminum, iron, and zinc ions. Examples of amine salts include salts with ammonia and organic nitrogenous bases strong enough to form salts with carboxylic acids.

As used herein, the term "therapeutically effective amount" means when applied to a compound of the invention is intended to denote an amount of the compound that is sufficient to ameliorate, palliate, stabilize, reverse, slow or delay the progression of a disorder or disease state, or of a symptom of the disorder or disease. In an embodiment, the method of the present invention provides for administration of combinations of compounds. In such instances, the "therapeutically effective amount" is the amount of a compound of the present invention in the combination sufficient to cause the intended biological effect.

As used herein, the term "treatment" or "treating" as used herein means ameliorating or reversing the progress or severity of a disease or disorder, or ameliorating or reversing one or more symptoms or side effects of such disease or disorder. "Treatment" or "treating", as used herein, also means to inhibit or block, as in retard, arrest, restrain, impede or obstruct, the progress of a system, condition or state of a disease or disorder. For purposes of this invention, "treatment" or "treating" further means an approach for obtaining beneficial or desired clinical results, where "beneficial or desired clinical results" include, without limitation, alleviation of a symptom, diminishment of the extent of a disorder or disease, stabilized (i.e., not worsening) disease or disorder state, delay or slowing of a disease or disorder state, amelioration or palliation of a disease or disorder state, and remission of a disease or disorder, whether partial or total.

In another embodiment, the compounds of Formula (I) are used for modulating the activity of a protein kinase c-abl.

As used herein, the term "modulating" or "modulation" refers to the alteration of the catalytic activity of a protein kinase. In particular, modulating refers to the activation or inhibition of the catalytic activity of a protein kinase, depending on the concentration of the compound or salt to which the protein kinase is exposed or, more preferably, the inhibition of the catalytic activity of a protein kinase. The term "catalytic activity" as used herein refers to the rate of phosphorylation of tyrosine, serine or threonine under the influence, direct or indirect, of a protein kinase.

The three main classes that pharmacological inhibitors of kinase activity are categorized by are (1) Type I, or "DFG-in" ATP competitive inhibitors, which directly compete with ATP in the ATP binding site (i.e., dual SRrc ABL inhibitor dasatinib, (2) Type II, or "DFG-out" ATP competitive inhibitors, which, in addition to binding the ATP binding site also engage an adjacent hydrophobic binding site that is only accessible when the kinase is in an inactivated configuration (i.e., the activation loop is oriented in a conformation that would block substrate binding) (i.e., imatinib, nilotinib), and (3) non-ATP competitive inhibitors that bind at sites outside the ATP binding site that affect the activity of the kinase (i.e., GNF-2).

As used herein, the phrase "compound(s) of this/the disclosure" includes any compound(s) of Formula (I), as well as clathrates, hydrates, solvates, or polymorphs thereof. And, even if the term "compound(s) of the disclosure" does not mention its pharmaceutically acceptable sat, the term includes salts thereof. In one embodiment, the compounds of this disclosure include stereochemically pure compounds, e.g., those substantially free (e.g., greater than 85% ee, greater than 90% ee, greater than 95% ee, greater than 97% ee, or greater than 99% ee) of other stereoisomers. That is, if the compounds of Formula (I) according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and/or acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

### Compounds of the present disclosure

The present disclosure provides compounds according to Formula (I): 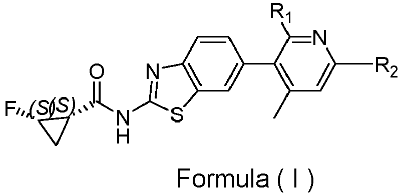 or a pharmaceutically acceptable salt thereof, wherein R₁ is -¹⁸F or - ¹¹CH₃ when R₂ is -H, or R₁ is -H when R₂ is -¹⁸F or -¹¹CH₃.

In one embodiment, the compound of Formula (I) is selected from the compound according to formula (IIA) and pharmaceutically acceptable salts thereof:

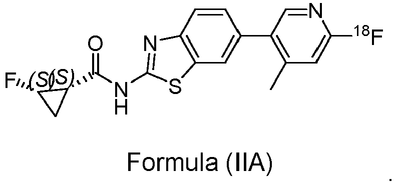

In one embodiment, the compound of Formula (I) is selected from the compound according to formula (IIB) and pharmaceutically acceptable salts thereof:

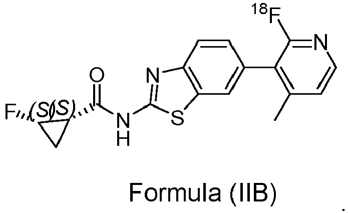

In yet another embodiment, there is provided a pharmaceutical composition comprising a therapeutically effective amount of a compound of Formula (I) or a pharmaceutically acceptable salt thereof, and a pharmaceutically acceptable carrier.

In another embodiment, there is provided a method for treating a neurodegenerative disease or disorder comprising administering to a subject in need thereof a therapeutically effective amount of a compound of Formula (I) or pharmaceutically acceptable salt thereof. That is, there is provided a medical use of the compound of Formula (I) or pharmaceutically acceptable salt thereof, wherein Formula (I) or pharmaceutically acceptable salt thereof is used as an active agent.

In one embodiment, the present disclosure relates to fluorine-18 labeled benzothiazole compounds as positron emission tomography (PET) tracers for imaging enzyme inhibitory activity comprising the compound and methods of using the compounds for diagnosis and imaging. In an embodiment, there is provided a method for treating a neurodegenerative disease comprising: administering to a subject in need thereof a therapeutically effective amount of the compound above or a pharmaceutically acceptable salt thereof.

In another embodiment, there is provided a method of determining an enzyme inhibitory activity, the method comprising: applying the compound above to a biological sample; and imaging the compound to determine the enzyme inhibitory activity. In various embodiments, the compound is used as a positron emission tomography (PET) tracer. The method can be a PET imaging method. Also, the method can be used in an AD-induced mouse AD model or an alpha-synuclein PFF-induced mouse PD model. The method can be used to determine c-abl upregulation or activation in a brain. In some embodiments, the method is for companion diagnosis for c-abl therapy or other disease-modifying agents as a predictive biomarker. The method can be used for a patient with a neurodegenerative disease.

### EXAMPLES

Hereinafter, the present disclosure is described in considerable detail with examples to help those skilled in the art understand the present disclosure.

The following examples are offered by way of illustration.

### Synthesis of Formula (I) Compound

### Example compound of the present disclosure is described in Scheme1

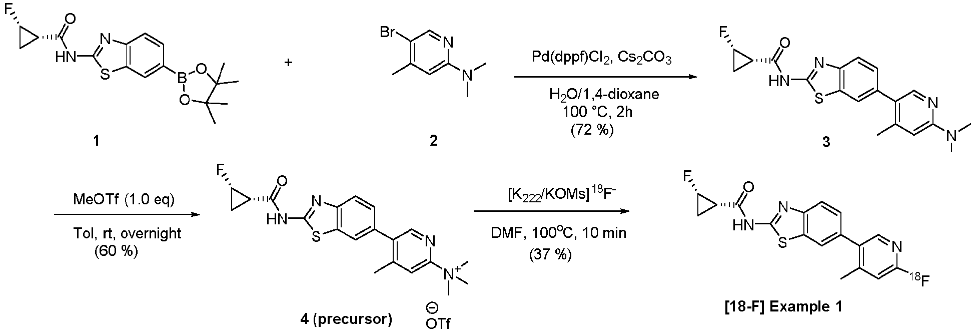

### Example 1. (1S,2S)-2-fluoro-N-(6-(6-(fluoro-18F)-4-methylpyridin-3-yl)benzo[d]thiazol-2-yl)cyclopropane-1-carboxamide

### Step1) (1S,2S)-N-(6-(6-(dimethylamino)-4-methylpyridin-3-yl)benzo[d] thiazol-2-yl)-2-fluorocyclopropane-1-carboxamide

To sealed tube was added **Compound 1** (906 mg, 2.5 mmol), **Compound 2** (430 mg, 2.0 mmol), (1,1'-bis(diphenylphosphino)ferrocene)-dichloropalladium(II) (44 mg, 0.06 mmol), cesium carbonate (39 mg, 0.12 mmol) and dioxane/water (6 mL/2 mL), then sealed and heated to 100 °C for 2 hours. The reaction mixture was cooled to RT and filtered through a pad of Celite, then extracted with EA. The combined organic layers were dried over Na₂SO₄, filtered and concentrated under reduced pressure to give a residue. The residue was purified by silica gel flash chromatography (EA: Hex = 3 : 1) to give **Compound 3** (Beige solid, yield 533 mg, 72%).

¹H NMR (400 MHz, CDCl₃) δ 8.06 (s, 1H), 7.76 - 7.72 (m, 2H), 7.36 (d, J = 8.3 Hz, 1H), 7.26 (s, 1H), 6.43 (s, 1H), 4.95 - 4.78 (m, 1H), 3.13 (s, 6H), 2.25 (s, 3H), 2.03 - 1.98 (m, 1H), 1.33 - 1.21 (m, 2H); LCMS (electrospray) m/z 371.16 (M+H)+.

### Step 2) 5-(2-((1S,2S)-2-fluorocyclopropane-1-carboxamido)benzo [d]thiazol-6-yl)-N,N,N,4-tetramethylpyridin-2-aminium trifluoromethanesulfonate

To a solution of **Compound 3** (110 mg, 0.30 mmol) in dry toluene (3 mL) added methyl trifluoromethanesulfonate (39 µL, 0.36 mmol). The mixture was stirred at RT overnight. The reaction mixture was concentrated and purified by silica gel flash chromatography (10% MeOH in DCM) to give **Compound 4** (white solid, yield 95 mg, 60%).

¹H NMR (400 MHz, DMSO-d6) δ 12.80 (NH, 1H), 8.51 (s, 1H), 8.10-8.05 (m 2H), 7.85 (d, J = 8.3 Hz, 1H), 7.48 (d, J = 8.3 Hz, 1H), 5.12 - 4.93 (m, 1H), 3.60 (s, 9H), 2.43 (s, 3H), 2.24 - 2.19 (m, 1H), 1.71-1.70 (m, 1H), 1.33 - 1.28 (m, 1H) ; ¹⁹F NMR (376 MHz, DMSO-d6) δ -77.74, -219.79;LCMS (electrospray) m/z 385.25 (M+H)+.

### Step 3) (1S,2S)-2-fluoro-N-(6-(6-(fluoro-¹⁸F)-4-methylpyridin-3-yl)benzo[d] thiazol-2-yl)cyclopropane-1-carboxamide

Aqueous [¹⁸F]fluoride (6.3 mCi) was passed through an anion-exchange resin (QMA). The [¹⁸F]fluoride was eluted to reaction vial using a solution of K222/KOMs complex 10 mg in ethanol (1 mL). The solution was concentrated to dryness by heating at 100 °C under N₂ gas. To the dried K222/K-[¹⁸F] complex was added **Compound 4,** precursor (5 mg) dissolved in 0.4 mL of DMSO. The solution was heated at 100 °C for 10 min and then **Example 1** was detected by radio-TLC (37%).

¹H NMR (400 MHz, DMSO-d*₆*) δ 12.76 (NH,1H), 8.118.12 (d, J=5.2 Hz, 1H), 8.01(s, 1H), 7.84 (d, J=8.4 Hz, 1H), 7.41-7.36 (m, 2H), 5.14 - 4.97 (m, 1H), 2.26-2.25 (m, 1H) ,2.25 (s, 3H), 1.79-1.72 (m, 1H), 1.33-1.29 (m, 1H); LCMS (electrospray) m/z 345.10 (M+H)+.

### Example 2. (1S,2S)-2-fluoro-N-(6-(2-(fluoro-¹⁸F)-4-methylpyridin-3-yl)benzo[d]thiazol-2-yl)cyclopropane-1-carboxamide

### Synthetic method is same as Example 1.

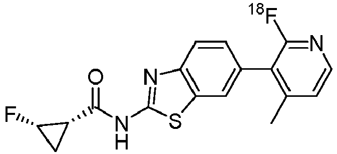

1H NMR (400 MHz, DMSO-d*₆*) δ 12.79 (NH,1H), 8.11 (s, 1H), 8.04 (s, 1H), 7.83 (d, J=8.4 Hz, 1H), 7.46 (d, J=8.4 Hz, 1H), 7.20(s, 1H), 5.14 - 4.97 (m, 1H), 2.34 (s, 3H), 2.26-2.22 (m, 1H), 1.79-1.72 (m, 1H), 1.33-1.29 (m, 1H); LCMS (electrospray) m/z 345.10(M+H)+.

## Claims

1. A compound of Formula (I), or pharmaceutically acceptable salt thereof: 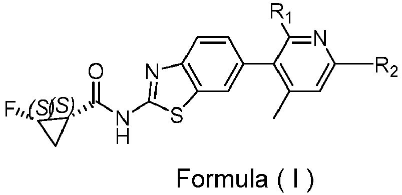 wherein
R₁ is -¹⁸F or -¹¹CH₃ when R₂ is -H, or
R₁ is -H when R₂ is -¹⁸F or -¹¹CH₃.

2. The compound of Claim 1, which is the compound of Formula (IIA) or a pharmaceutically acceptable salt thereof:

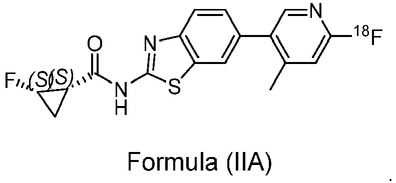

3. The compound of Claim 1, which is the compound of formula (IIB) or a pharmaceutically acceptable salt thereof:

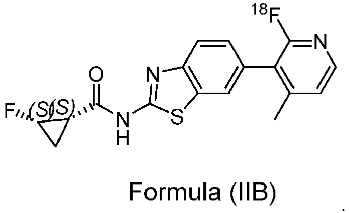

4. A pharmaceutical composition comprising a therapeutically effective amount of the compound of Claim 1 or a pharmaceutically acceptable salt thereof, and a pharmaceutically acceptable carrier.

5. A compound of Claim 1 or a pharmaceutically acceptable salt thereof for use in a method for treating a neurodegenerative disease, the method comprising:
administering to a subject in need thereof a therapeutically effective amount of the compound of Claim 1 or a pharmaceutically acceptable salt thereof.

6. A compound of Claim 1 or a pharmaceutically acceptable salt thereof for use in a method of determining an enzyme inhibitory activity, the method comprising:
applying the compound of Claim 1 to a biological sample; and
imaging the compound to determine the enzyme inhibitory activity.

7. An *in vitro* method of determining an enzyme inhibitory activity, the method comprising:
applying the compound of Claim 1 to a biological sample; and
imaging the compound to determine the enzyme inhibitory activity.

8. The compound for use of Claim 6 or *in vitro* method of Claim 7, wherein the compound is used as a positron emission tomography (PET) tracer.

9. The compound for use of Claim 6 or *in vitro* method of Claim 7, which is a PET imaging method.

10. The compound for use of Claim 6 or *in vitro* method of Claim 7, wherein the method is used in an AD-induced mouse AD model or an alpha-synuclein PFF-induced mouse PD model.

11. The compound for use of Claim 6 or *in vitro* method of Claim 7, wherein the method is used to determine c-abl upregulation or activation in a brain.

12. The compound for use of Claim 6 or *in vitro* method of Claim 7, wherein the method is for companion diagnosis for c-abl therapy or other disease-modifying agents as a predictive biomarker.

13. The compound for use of Claim 6 or *in vitro* method of Claim 7, wherein the method is used for a patient with a neurodegenerative disease.

## Patentansprüche

1. Verbindung der Formel (I) oder pharmazeutisch verträgliches Salz davon: 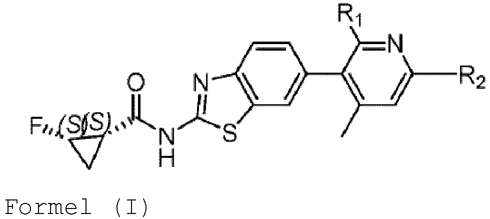 wobei
R₁ -¹⁸F oder -¹¹CH₃ ist, wenn R₂ -H ist, oder
R₁ -H ist, wenn R₂ -¹⁸F oder -¹¹CH₃ ist.

2. Verbindung nach Anspruch 1, welche die Verbindung der Formel (IIA) oder ein pharmazeutisch verträgliches Salz davon ist:

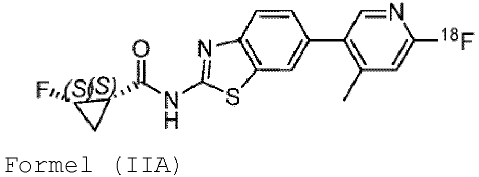

3. Verbindung nach Anspruch 1, welche die Verbindung der Formel (IIB) oder ein pharmazeutisch verträgliches Salz davon ist:

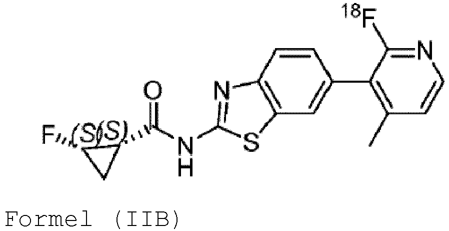

4. Pharmazeutische Zusammensetzung, umfassend eine therapeutisch wirksame Menge der Verbindung nach Anspruch 1 oder ein pharmazeutisch verträgliches Salz davon und einen pharmazeutisch verträglichen Träger.

5. Verbindung nach Anspruch 1 oder ein pharmazeutisch verträgliches Salz davon zur Verwendung in einem Verfahren zum Behandeln einer neurodegenerativen Erkrankung, wobei das Verfahren Folgendes umfasst:
Verabreichen einer therapeutisch wirksamen Menge der Verbindung nach Anspruch 1 oder eines pharmazeutisch verträglichen Salzes davon an ein Subjekt, das dessen bedarf.

6. Verbindung nach Anspruch 1 oder ein pharmazeutisch verträgliches Salz davon zur Verwendung in einem Verfahren zum Bestimmen einer Enzyminhibitoraktivität, wobei das Verfahren Folgendes umfasst:
Anwenden der Verbindung nach Anspruch 1 auf eine biologische Probe; und
Abbilden der Verbindung zum Bestimmen der Enzyminhibitoraktivität.

7. *In-vitro*-Verfahren zum Bestimmen einer Enzyminhibitoraktivität, wobei das Verfahren Folgendes umfasst:
Anwenden der Verbindung nach Anspruch 1 auf eine biologische Probe; und
Abbilden der Verbindung zum Bestimmen der Enzyminhibitoraktivität.

8. Verbindung zur Verwendung nach Anspruch 6 oder *In-vitro-*Verfahren nach Anspruch 7, wobei die Verbindung als Positronen-Emissions-Tomografie(PET)-Tracer verwendet wird.

9. Verbindung zur Verwendung nach Anspruch 6 oder *In-vitro-*Verfahren nach Anspruch 7, welches ein PET-Bildgebungsverfahren ist.

10. Verbindung zur Verwendung nach Anspruch 6 oder *In-vitro-*Verfahren nach Anspruch 7, wobei das Verfahren in einem ADinduzierten AD-Modell in der Maus oder einem alpha-Synuclein-PFF-induzierten-PD-Modell in der Maus verwendet wird.

11. Verbindung zur Verwendung nach Anspruch 6 oder *In-vitro-*Verfahren nach Anspruch 7, wobei das Verfahren verwendet wird, um eine c-abl-Hochregulierung oder Aktivierung in einem Gehirn zu bestimmen.

12. Verbindung zur Verwendung nach Anspruch 6 oder *In-vitro-*Verfahren nach Anspruch 7, wobei das Verfahren zur Begleitdiagnostik für eine c-abl-Therapie oder andere krankheitsmodifizierende Mittel als prädiktiver Biomarker dient.

13. Verbindung zur Verwendung nach Anspruch 6 oder *In-vitro-*Verfahren nach Anspruch 7, wobei das Verfahren für einen Patienten mit einer neurodegenerativen Erkrankung verwendet wird.

## Revendications

1. Composé de formule (I), ou sel pharmaceutiquement acceptable de celui-ci : 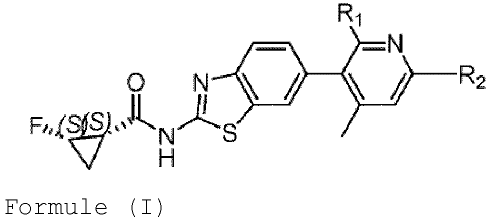 dans lequel
R₁ est -¹⁸F ou -¹¹CH₃ lorsque R₂ est -H, ou
R₁ est -H lorsque R₂ est -¹⁸F ou -¹¹CH₃.

2. Composé selon la revendication 1, qui est le composé de formule (IIA) ou un sel pharmaceutiquement acceptable de celui-ci :

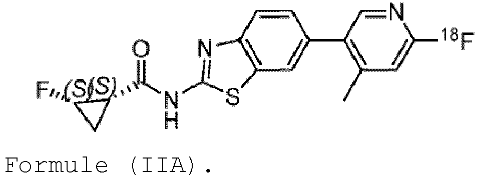

3. Composé selon la revendication 1, qui est le composé de formule (IIB) ou un sel pharmaceutiquement acceptable de celui-ci :

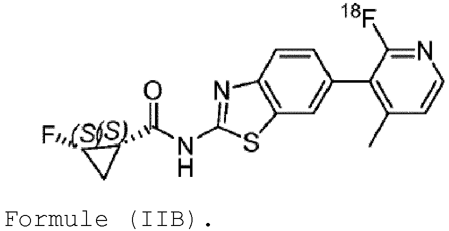

4. Composition pharmaceutique comprenant une quantité thérapeutiquement efficace du composé selon la revendication 1 ou d'un sel pharmaceutiquement acceptable de celui-ci, et un véhicule pharmaceutiquement acceptable.

5. Composé selon la revendication 1, ou un sel pharmaceutiquement acceptable de celui-ci, destiné à être utilisé dans un procédé de traitement d'une maladie neurodégénérative, le procédé comprenant :
l'administration, à un sujet en ayant besoin, d'une quantité thérapeutiquement efficace du composé selon la revendication 1 ou d'un sel pharmaceutiquement acceptable de celui-ci.

6. Composé selon la revendication 1, ou un sel pharmaceutiquement acceptable de celui-ci, destiné à être utilisé dans un procédé de détermination d'une activité inhibitrice enzymatique, le procédé comprenant :
l'application du composé selon la revendication 1 à un échantillon biologique ; et
l'imagerie du composé pour déterminer l'activité inhibitrice enzymatique.

7. Procédé *in vitro* de détermination d'une activité inhibitrice enzymatique, le procédé comprenant :
l'application du composé selon la revendication 1 à un échantillon biologique ; et
l'imagerie du composé pour déterminer l'activité inhibitrice enzymatique.

8. Composé destiné à être utilisé selon la revendication 6 ou procédé *in vitro* selon la revendication 7, dans lequel le composé est utilisé comme traceur de tomographie par émission de positrons (PET).

9. Composé destiné à être utilisé selon la revendication 6 ou procédé *in vitro* selon la revendication 7, qui est un procédé d'imagerie par PET.

10. Composé destiné à être utilisé selon la revendication 6 ou procédé *in vitro* selon la revendication 7, dans lequel le procédé est utilisé dans un modèle d'AD de souris induit par AD ou un modèle de PD de souris induit par alpha-synucléine PFF.

11. Composé destiné à être utilisé selon la revendication 6 ou procédé *in vitro* selon la revendication 7, dans lequel le procédé est utilisé pour déterminer la régulation positive ou l'activation de c-abl dans un cerveau.

12. Composé destiné à être utilisé selon la revendication 6 ou procédé *in vitro* selon la revendication 7, dans lequel le procédé est destiné à un diagnostic compagnon pour une thérapie par c-abl ou d'autres agents modificateurs de maladies en tant que biomarqueur prédictif.

13. Composé destiné à être utilisé selon la revendication 6 ou procédé *in vitro* selon la revendication 7, dans lequel le procédé est utilisé pour un patient atteint d'une maladie neurodégénérative.
